(19) Europäisches Patentamt
European Patent Office
Office européen des brevets

(11) Publication number: 0 458 519 A2

# (12) EUROPEAN PATENT APPLICATION

(21) Application number: 91304378.2

(51) Int. Cl.[5]: C07H 19/04, A61K 31/70

(22) Date of filing: 15.05.91

(30) Priority: 24.05.90 GB 9011623

(43) Date of publication of application: 27.11.91 Bulletin 91/48

(84) Designated Contracting States: AT BE CH DE DK ES FR GB GR IT LI LU NL SE

(71) Applicant: EFAMOL HOLDINGS PLC
Efamol House Woodbridge Meadows
Guildford Surrey GU1 1BA (GB)

(72) Inventor: Reese, Colin B., c/o Department of Chemistry
King's College London, University of London
Strand, London WC2R 2LS (GB)
Inventor: Buck, Ildiko, c/o Department of Chemistry
King's College London, University of London
Strand, London WC2R 2LS (GB)

(74) Representative: Miller, Joseph et al
J. MILLER & CO. Lincoln House 296-302 High Holborn
London WC1V 7JH (GB)

## (54) Synthesis of nucleotide and nucleoside derivatives.

(57) A method of synthesis of a substituted-amine derivative of a nucleoside or nucleotide wherein the substituent amino group is introduced by a nucleophilic displacement reaction between a sulphone or sulphoxide substituted heterocyclic compound forming or to form the heterocyclic moiety of said derivative and an alpha-amino acid or other reactive amino compound.

EP 0 458 519 A2

This invention relates to synthesis of nucleotide and nucleoside derivatives.

The nucleotide adenylosuccinic acid (*1*) has been suggested as valuable in the treatment of Duchenne muscular dystrophy and other conditions. It is believed to assist in improving the energy supply to the muscle. As such it is likely to be of benefit in any diseases in which impaired function of skeletal muscle, cardiac muscle or smooth muscle, or of nerves in the central or peripheral nervous system is important. It is available by enzymatic synthesis but an unambiguous chemical synthesis of it, as such or in the nucleoside form herein referred to as adenosylosuccinic acid (*3*), has been desirable.

We have found such a synthesis, the key step in the specific application being a nucleophilic displacement reaction between a purine, carrying a sulphoxide or sulphone group at the 6-position, and L-aspartic acid, conveniently for example in the form of the dibenzyl ester. Conveniently the purine already carries a 9-(β-D-furanosyl) group, but equally the displacement reaction may be carried out first. Conveniently also, but not essentially, the phosphate group of the final nucleotide derivative is attached after the synthesis of the nucleoside structure.

The invention most broadly extends to a method of synthesis of a substituted-amine derivative of a nucleoside or nucleotide wherein the substituent amino group is introduced by a nucleophilic displacement reaction between a sulphone or sulphoxide substituted heterocyclic compound forming or to form the heterocyclic moiety of said derivative and an alpha-amino acid or other reactive amino compound.

Specifically the invention provides a method for production of adenosylo-succinic acid (*3*) or adenylo-succinic acid (*1*) wherein the succinate group is attached to the purine ring by displacement reaction between L-aspartic acid and a purine carrying a sulphoxide or sulphone group at the 6-position.

The product acids are of course also an aspect of the invention and when prepared for therapeutic use may be in the free form or in salt, ester, amide or other form giving rise in the body to the acid, as measured by plasma or tissue level rise in its level in free form or as other, bodily synthesised, derivatives. References to the acids herein, particularly as the products of the method of the invention in the claims, thus include reference to them in such form.

Suitably the sulphoxide or sulphone substituted purine as the immediate starting material has been prepared by oxidation of the corresponding thio-derivative.

Suitably further the thio-derivative has been prepared by reaction of a thiol with the heterocyclic compound as a chloro or other reactive derivative. It is known for example to prepare 6-chloro-9-(β-D-ribofuranosyl) purine as its 2′,3′,5′-triacetate, see Gerster et al. J. Org. Chem. (1963) 28, 945, and Robins et al "Nucleic Acid Chemistry", Part 3, pages 144 to 148, (Wiley Interscience, New York, eds Townsend and Tipson, 1986).

During the synthesis non-reacting groups may be present as derivatives, for example to prevent undesired reaction or to ease subsequent purification in a manner per se familiar. In the reaction with the thiol the pentose when present may for example be in the form of its triacetate. Similarly, in oxidation of the thio derivative to the sulphoxide or sulphone, a suitable form of the pentose is the 2′,3′-O-cyclopentylidene or isopropylidene derivative. The presence of such protection is for example convenient in subsequent 5′-phosphorylation, though under suitable conditions phosphorylation at the 5′-position, where the alcohol is primary, can be selective over the 2′ and 3′ (secondary) alcohol groups; in particular enzymatic phosphorylation is suitable. The aspartic acid used in the preparation of adenosylosuccinic acid or adenylo-succinic acid is conveniently a derivative, for example the dibenzyl ester. No restriction to any particular derivative in any of these instances is however necessary or intended.

Similarly a suitable sulphoxide or sulphone group is an aromatic particularly carbocyclic group with electron withdrawing substituents for example a p-chloro or p-bromo phenyl sulphoxide or sulphone, but no restriction to any particular group or electron withdrawing substituent or position is intended and o-, m-, or p-nitro phenyl,2,5-dichloro phenyl and 2,4,5-trichloro phenyl groups are further examples. The sulphoxides are better, requiring milder conditions will less risk of racemisation, but sulphones are as effective with more strongly electron withdrawing substituents.

While the invention lies primarily in the synthesis of nucleosides or nucleotides such as adenosylo- or adenylo-succinic acid it also extends to their use in treatment or in the preparation of medicaments. The adenylosuccinic acid is the primarily useful compound in treatment, but administration of adenosylosuccinic acid with subsequent bodily phosphorylation is not excluded. The indicated conditions for the specific compounds are of muscular weakness, particularly in the muscular dystrophies of various types, in cardiac disorders associated with myocardial weakness or failure or in other disorders associated with weakness of skeletal, cardiac or smooth muscles such as diabetes, chronic fatigue syndrome or postviral fatigue syndrome or disorders of urinary tract or gastrointestinal or other smooth muscle function; also degenerative and other diseases of the central and peripheral nervous system such as multiple sclerosis, Alzheimer's disease and other dementias, impaired intellectual development or intellectual deterioration from any cause and peripheral neuropathies of any cause.

Full detail of a particular synthesis according to the invention is given below.

In summary 6-(4-chlorophenylthio)-9-(2,3-O-cyclopentylidene-β-D-ribofuranosyl)purine is prepared from 6-chloro-9-(2,3,5-tri-O-acetyl-β-D-ribofuranosyl)purine in three steps and converted, by treatment with 3-chloroperbenzoic acid, into the corresponding sulphoxide and crystalline sulphone in ca. 88 and 65% isolated yields, respectively. When the sulphoxide is heated with dibenzyl L-aspartate in N,N-dimethyl-acetamide solution at 70 to 75°C for 28 hours, dibenzyl N-[9-(2,3-O-cyclopentylidene-β-D-ribofuranosyl)purin-6-yl]-L(*11*) aspartate is obtained in ca. 70% isolated yield. Removal of the protecting groups gives adenosylosuccinic acid, N-[9-(β-D-ribofuranosyl)purin-6-yl]-L aspartic acid (*3*) as a crystalline solid in ca. 68% yield; phosphorylation of (*11*) with dibenzyl phosphorochloridate and removal of the protecting groups gives adenylosuccinic acid N-[9-(5-phospho-β-D-ribofuranosyl)purin-6-yl]-L aspartic acid (*1*), isolated as its ammonium salt, in ca. 66% yield. When the somewhat less reactive sulphone is similarly converted the products obtained appear to be contaminated with diastereoisomeric impurities, but separation of the relatively small amounts of contaminant isomer can follow, e.g. by crystallisation in the case of the nucleosides or if the compound is not crystalline (as generally with the nucleotides) then by chromatography under non-isomerising conditions.

The details of the above synthesis and its background follow:-

Despite the fact that {N-[9-(β-D-ribofuranosyl) purin-6-yl]-L-aspartic acid} 5′-phosphate [adenylosuccinic acid (*1*)] has been known for a long time to be the intermediate in the biosynthetic conversion of inosine 5′-phosphate into adenosine 5′-phosphate (*2*), we are unaware of any previous report relating to its chemical synthesis. However, an enzymatic synthesis of adenylosuccinic acid (*1*), involving the adenylosuccinase-promoted addition of fumarate to the amino function of adenosine 5′-phosphate (*2*), has been reported. There are two accounts in the literature relating to the chemical synthesis of N-[9-(β-D-ribofuranosyl)purin-6-yl]-L-aspartic acid (*3*),the nucleoside component of adenylosuccinic acid (*1*). The first synthesis, which presumably led to a mixture of (*1*) and its diastereo-isomer (see below) derived from D-aspartic acid, was effected by heating unprotected 6-methylmercapto-9-(β-D-ribofuranosyl)purine (*4a*) together with unprotected D,L-aspartic acid in dilute aqueous sodium hydroxide solution, and led to only a very small quantity of isolated product. The second synthesis, which involved heating unprotected 6-chloro-9-(β-D-ribofuranosyl)purine (*4b*) and L-aspartic acid together under similar conditions, was also carried out on a small scale. As (*3*) is a polar, ionizable compound, anion-exchange chromatography was used in the course of its purification. Ion-exchange chromatography was similarly used in the purification of enzymatically-synthesized adenylosuccinic acid (*1*). It seemed to us that if the large scale preparation of (*1*) and (*3*) was to be a feasible proposition, it would be desirable to work with protected intermediates which were soluble in organic solvents and which would be purified by standard techniques (i.e. chromatography on silica gel), and to choose protecting groups which could be removed cleanly under mild conditions. We have followed this strategy and we now report the unambiguous chemical synthesis of the ammonium salt of adenylosuccinic acid (*1*) and its constituent nucleoside (*3*).

6-Chloro-9-(2,3,5-tri-O-acetyl-β-D-ribofuranosyl) purine (*5*), which was prepared from inosine by the two step literature procedure

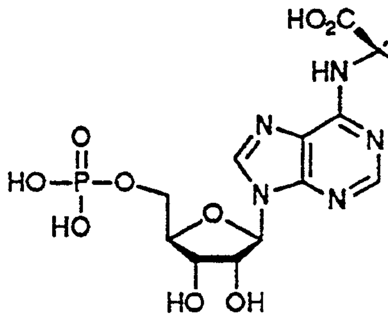

(1)

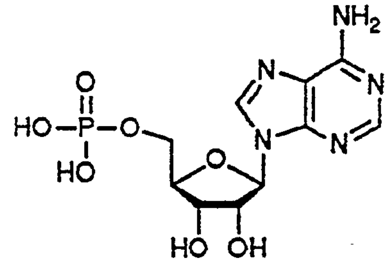

(2)

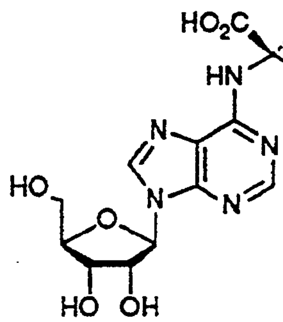

(3)

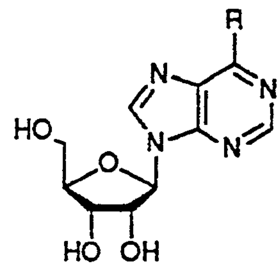

(4) a; R = SMe
b; R = Cl

in virtually quantitative yield, was allowed to react with a slight excess each of 4-chlorothiophenol and triethylamine in methanol solution at room temperature for 30 minutes to give the corresponding 6-(4-chlorophenylthio)-derivative (*6a*). The latter compound was deacetylated by treatment with ammonia in methanol solution to give (*6b*) in ca. 72% overall yield. When (*6b*) was heated with an excess of 1,1-dimethoxycyclopentane in the presence of 0.1 mol. equiv. of toluene-4-sulphonic acid monohydrate in tetrahydrofuran solution, under nitrogen, at 50°C for 1 hour, the corresponding 2′,3′-O-cyclopentylidene derivative (*7*) was obtained and was isolated as a colourless foam in ca. 84% yield. Treatment of (*7*) with ca. 3.0 mol. equiv. of 3-chloroperbenzoic acid in dichloromethane solution at room temperature for 3 hours gave the corresponding sulphone (*8*) which was isolated as a colourless crystalline solid in 65% yield. When (*7*) was allowed to react with ca. 1.05 mol. equiv. of 3-chloroperbenzoic acid at -5°C, the corresponding sulphoxide (*9*) was obtained and was isolated as a colourless foam in ca. 88% yield. It was easily possible to distinguish between the sulphone (*8*) and the slightly more polar sulphoxide (*9*) by t.l.c. and by $^{13}C$ n.m.r. spectroscopy. It was indeed clear from its $^{13}C$ n.m.r. spectrum that (*9*) itself was, as expected, a mixture of diasteroisomers; both are active in the displacement reaction to give the desired product.

Purine nucleoside 6- and 8-sulphone derivatives have been shown to be particularly susceptible to nucleophilic attack. However, although the sulphone (*8*) was found to react readily with cyclohexylamine at room temperature, stronger conditions were required to effect its reaction with esters of aspartic acid. Thus, it was necessary to heat (*8*) with ca. 2.5 mol. equiv. of dibenzyl L-aspartate [generated from its

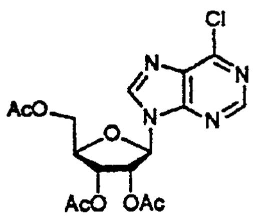

(5)

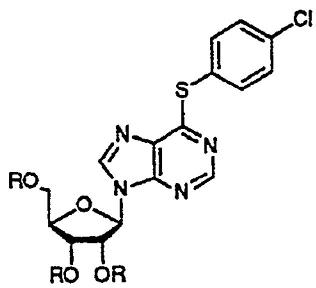

(6) a; R = Ac
b; R = H

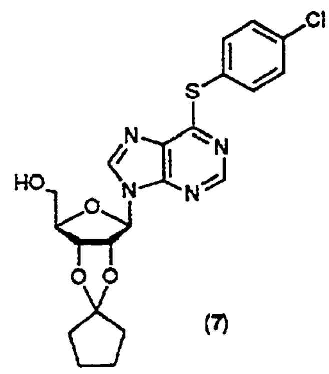

(7)

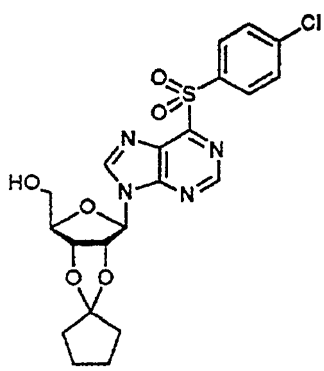

(8)

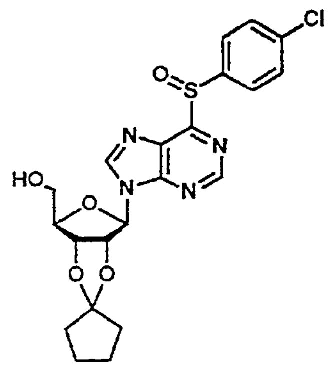

(9)

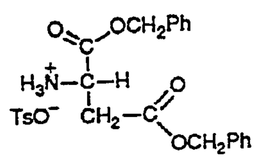

(10)

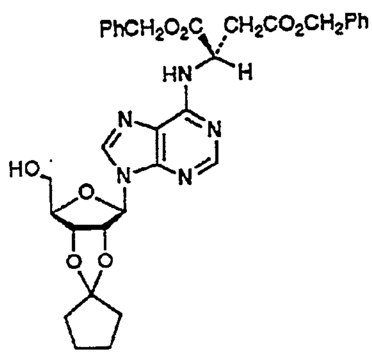

(11)

toluene-4-sulphonate salt (*10*) and di-isopropylethyl-amine] in N,N-dimethylacetamide solution at 100°C for 24 hours to effect its conversion to dibenzyl N-[9-(2, 3-O-cyclopentylidene-β-D-ribofuranosyl)purin-6-yl]-L-aspartate (*11*). The latter compound (*11*), which was later shown (see below) to be contaminated with a diastereoisomer, was isolated from the products in 60% yield. The corresponding sulphoxide (*9*) proved to be somewhat more susceptible to nucleophilic attack by the amino function of dibenzyl L-aspartate. Thus when (*9*) was heated with an excess each of (*10*) and di-isopropyl-ethylamine in N,N-dimethylacetamide solution at 70 to 75°C, the reaction proceeded almost to completion in 28 hours, and the desired product (*11*) was obtained in ca. 70% isolated yield. Furthermore, this material was later shown (see below) to be virtually free from a diastereoisomeric contaminant.

The protecting groups were removed from a sample of dibenzyl N-[9-(2,3-O-cyclopentylidene-β-D-ribofuranosyl)purin-6-yl]-L-aspartate (*11*), prepared from the intermediate sulphoxide (*9*), by a two step process. The cyclopentylidene group was first removed by treatment with formic acid-water (3:2 v/v) at room temperature for 3 hours, and the two benzyl groups were then removed by hydrogenolysis in the presence of palladized charcoal in ethanol - acetic acid - water (8:1:1 v/v), also at room temperature. The desired N-[9-(β-D-ribofuranosyl)purin-6-yl]-L-aspartic acid (*3*) was isolated from the products as a pure crystalline solid in 68% yield, based on (*11*). When the protected intermediate (*11*), prepared from the sulphone (*8*), was unblocked in the same way, the unprotected nucleoside (*3*) was obtained in 61% isolated yield. Following the crystallization of the latter product from aqueous ethanol, examination of the remaining mother liquors by l.c. revealed, in addition to (*3*) [ca. 66%], the presence of a longer $R_T$ product [ca. 33%] believed (see below) to be its diastereoisomer (*12*). However, l.c. analysis of the crystalline material prepared from the sulphone (*8*) revealed that it was virtually pure (*3*), which was only very slightly (<2%) contaminated with its diastereoisomer (*12*). Evidence for structure of the latter by-product was obtained by allowing the sulphone intermediate (*8*) to react with the racemic modification of dibenzyl aspartate at 100°C for 24 hours. Following the removal of the protecting groups from the purified products, a 1:1 mixture [as indicated by l.c.] of (*3*) and (*12*) was obtained in 36% overall yield, based on (*8*). The $R_T$ 's of the major and minor components present in the above mother liquors corresponded to the $R_T$ 's of (*3*) and (*12*), respectively, observed in the course of the l.c. examination of the latter 1:1 mixture.

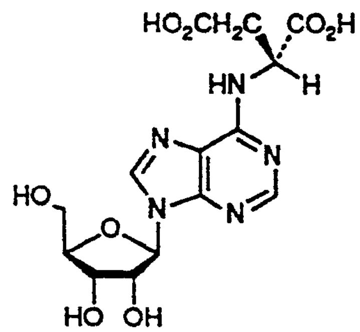

(12)

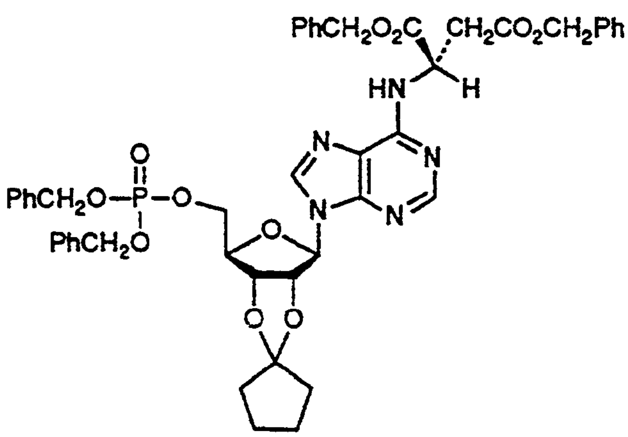

(13)

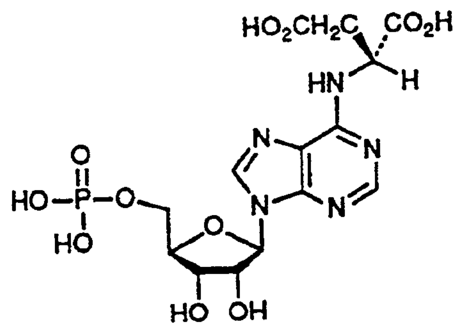

**(14)**

Dibenzyl N-[9-(2,3-O-cyclopentylidene-β-D-ribofuranosyl)purin-6-yl]-L-aspartate (*11*), prepared from the sulphoxide (*9*), was then allowed to react with ca. 3.0 mol. equiv. of dibenzyl phosphorochloridate in anhydrous pyridine solution at -40°C for 2 hours to give, following short column chromatography of the products on silica gel, the corresponding fully protected 5′-phosphate (*13*). The protecting groups were removed from the latter compound (*13*) by the two step unblocking procedure described above in the conversion of (*11*) into (*3*). An excess of concentrated aqueous ammonia was added to a solution of the resulting adenylosuccinic acid (*1*) in water. The solution obtained was then concentrated under reduced pressure to small volume and added to ca. one hundred times its volume of absolute ethanol to give the ammonium salt of adenylosuccinic acid (*1*) as a colourless solid precipitate in ca. 66% overall yield for the three steps starting from (*11*). The latter material was found by l.c. to be ca. 99.5% pure, and to have the same $R_T$ as a commercial sample of what was assumed to be enzymatically-synthesized adenylosuccinic acid (*1*); it was further characterised on the basis of its $^1H$, $^{13}C$ and $^{31}P$ n.m.r. spectra, and was also found to undergo quantitative conversion to adenosine 5′-phosphate (*2*) when it was treated with adenylosuccinate lyase. A sample of dibenzyl N-[9-(2,3-O-cyclopentylidene-β-D-ribofuranosyl)purin-6-yl]-L-aspartate (*11*), prepared from the sulphone (*8*), was also converted into the ammonium salt of adenylosuccinic acid (*1*). The latter product, which was obtained from (*11*) in 59% overall yield by the same three step procedure, was found by l.c. analysis to consist of adenylosuccinate (ca. 91%) and a shorter $R_T$ component (9%), believed to be the ammonium salt of its diastereoisomer (*14*). A small quantity of this shorter $R_T$ material was isolated by preparative l.c., and was then treated with bacterial alkaline phosphatase to give what appeared from l.c. analysis to be the diastereoisomeric impurity (*12*) found in samples of N-[9-β-D-ribofuranosyl)purin-6-yl]-L-aspartic acid (*3*), prepared from the sulphone (*8*).

We believe that the chemical synthesis of adenylosuccinic acid (*1*), starting from the sulphoxide (*9*), is superior to the enzymatic synthesis and it would appear that our strategy (see above) has been successful: the fully protected intermediate (*13*) is freely soluble in organic solvents and can be purified by conventional chromatographic techniques. Furthermore, the latter intermediate (*13*) can then be cleanly unblocked under relatively mild conditions to give virtually pure adenylosuccinic acid (*1*) without any further purification steps. It would therefore seem likely that the present synthesis could readily be scaled-up if large quantities of adenylosuccinic acid (*1*) were needed, for example, for the treatment of sufferers from Duchenne muscular dystrophy. The enzymatic synthesis of (*1*) necessarily leads to an unprotected product. Although, as indicated above, the latter material may be purified by anion-exchange chromatography, it is quite possible that the scalingup of this process would present considerable practical difficulties. Finally, the unambiguous chemical synthesis of adenylosuccinic acid (*1*) from dibenzyl L-aspartate and the direct comparison of the chemically-with enzymatically-synthesized material proves beyond doubt that the absolute configuration of C-2 of the succinate residue in adenylosuccinic acid (*1*) is S.

## EXPERIMENTAL

$^1H$ and $^{13}C$ N.m.r. spectra were measued at 360 and 90.6 MHz, respectively, with a Bruker AM 360 spectrometer; tetramethylsilane was used as an internal standard. Merck silica gel H was used for short column chromatography. Merck silica gel 60 $F_{254}$ t.l.c. plates were developed in solvent system A [$CHCl_3$-EtOH (95:5 v/v)] or B [$CHCl_3$-EtOH (98:2 v/v)] unless otherwise stated. L.c. was carried out on a Jones APEX ODS 5μ column, which was eluted isocratically with mixtures of 0.1 M - triethylammonium acetate and acetonitrile. Pyridine, di-isopropylethylamine and benzene were dried by heating, under reflux, with calcium hydride and were then distilled; tetrahydrofuran was dried by heating, under reflux, first with sodium benzophenone and then with lithium aluminium hydride; N,N-dimethylacetamide was dried over no. 4A molecular sieves. The toluene-4-sulphonate salt of dibenzyl L-aspartate, adenylosuccinic acid and enzymes were purchased from the Sigma Chemical Co. Ltd.

**6-Chloro-9-(2,3,5-tri-O-acetyl-β-D-ribofuranosyl) purine (*5*).**

Acetic anhydride (145 ml, 1.54 mol) was added to a stirred suspension of inosine (14.5 g, 54.1 mmol) in anhydrous pyridine (175 ml). The reactants were maintained at 40°C for 1 hour, and the resulting clear solution was evaporated under reduced pressure. After the colourless solid residue obtained had been triturated with anhydrous ether (75 ml), it was filtered, washed with ether (2 x 75 ml) and dried in vacuo at 50°C; yield 21.1 g; $R_F$ 0.20 (system A).

The latter material (12.0 g) was added in small portions to a solution of freshly distilled phosphorus oxychloride (60.0 ml, 0.64 mol) and N,N-dimethylaniline (4.0 ml, 25.1 mmol). The resulting suspension was stirred under nitrogen and immersed in a pre-heated (to 115°C) oil-bath. After ca. 4 minutes, a clear homogeneous solution, which started to boil, was obtained. After a further period of 3 minutes, the products were concentrated under reduced pressure (bath temperature ca. 40°C). The residue was cooled (ice-bath) and crushed ice (ca. 100 g) was added. The stirred mixture was maintained at 0°C (ice-bath) for 1 hour, and was then extracted with chloroform (3 x 75 ml). After the combined organic extracts had been washed first with M-hydrochloric acid (5 x 70 ml) and then with water (2 x 70 ml), they were dried ($MgSO_4$) and evaporated under reduced pressure. Repeated evaporation of the residue from chloroform gave the title compound as a pale yellow foam; yield 12.6 g (almost quantitative yield for the two steps, starting from inosine); $R_F$ 0.43 (system A), 0.28 (system B); $\delta_H$ ($CDCl_3$) 2.10 (3H, s), 2.14 (3H, s), 2.17 (3H, s), 4.35 - 4.55 (3H, m), 5.66 (1H, t, J 5.1 Hz), 5.96 (1H, t, J 5.3 Hz), 6.25 (1H, d, J 5.1 Hz), 8.33 (1H, s), 8.79 (1H, s); $\delta_c$ ($CDCl_3$): 20.29, 20.44, 20.66, 62.81, 70.37, 73.02, 132.26, 143.56, 151.16, 151.53, 152.23, 169.28, 169.49, 170.19.

**6-(4-Chlorophenylthio)-9-(2,3,5-tri-O-acetyl-β-D-ribofuranosyl)purine (*6a*).**

4-Chlorothiophenol (0.87 g, 6.0 mmol) and triethylamine (0.84 ml, 6.0 mmol) were added to a stirred solution of 6-chloro-9-(2,3,5-tri-O-acetyl-β-D-ribofuranosyl)purine (2.06 g, ca. 5.0 mmol) in methanol (35 ml) at room temperature. After 30 minutes, the products were concentrated under reduced pressure and the residue was partioned between chloroform (200 ml) and saturated aqueous sodium hydrogen carbonate (100 ml). The dried ($MgSO_4$) organic layer was concentrated under reduced pressure and the residue was chromatographed on silica gel. Fractions (eluted with $CHCl_3$) containing the desired product were combined and evaporated under reduced pressure to give the title compound as a colourless glass (2.27 g; ca. 87% yield); $R_F$ 0.50 (system A), 0.32 (system B); $\delta_H$ ($CDCl_3$) 2.09 (3H, s), 2.13 (3H, s), 2.15 (3H, s), 4.38 (1H, dd, J 5.1, 12.9 Hz), 4.42 - 4.50 (2H, m), 5.66 (1H, t, J 5.0 Hz), 5.96 (1H, t, J 5.3 Hz), 6.22 (1H, d, J 5.2 Hz), 7.45 (2H, d, J 8.3 Hz), 7.59 (2H, d, J 8.3 Hz), 8.18 (1H, s), 8.63 (1H, s); $\delta_C$ ($CDCl_3$) 20.40, 20.54, 20.78, 62.97, 70.57, 73.10, 80.43, 86.53, 125.40, 129.61, 131.36, 136.10, 136.87, 141.58, 148.60, 152.52, 160.82, 169.34, 169.58, 170.31.

**6-(4-Chlorophenylthio)-9-β-D-ribofuranosyl)purine (*6b*).**

(a) 6-(4-Chlorophenylthio)-9-(2,3,5-tri-O-acetyl-β-D-ribofuranosyl)purine (2.27 g, ca. 4.4. mmol) was dissolved in 8 M-methanolic ammonia (20 ml) and the solution was allowed to stand at room temperature overnight. The products were concentrated under reduced pressure and the residue was purified by short column chromatography on silica gel. The appropriate fractions, eluted with $CHCl_3$-EtOH (95:5 v/v), were combined and evaporated under reduced pressure to give the title compound as colourless glass (1.67 g, ca. 97%); $R_F$ 0.24 (system A), 0.05 (system B); $\delta_H$ [$(CD_3)_2SO$] 3.58 (1H, m), 3.70 (1H, m), 3.99 (1H, m), 4.19 (1H, m), 4.60 (1H, m), 5.12 (1H, t, J 5.5 Hz), 5.25 (1H, d, J 5.0 Hz), 5.55 (1H, d, J 5.9 Hz), 6.01 (1H, d, J 5.5 Hz), 7.57 (2H, d, J 8.4 Hz), 7.67 (2H, d, J 8.4 Hz), 8.61 (1H, s), 8.79 (1H, s); $\delta_C$[$(CD_3)_2$ SO] 61.13, 70.17, 73.81, 85.65, 87.86, 125.63, 129.35, 130.56, 134.67, 137.16, 143.81, 148.66, 151.62, 158.46, 171.57.

(b) The above experiment was repeated on a larger scale by allowing a solution of 6-(4-chlorophenylthio)-9-(2,3,5-tri-O-acetyl-β-D-ribofuranosyl)purine (26.85 g, ca. 51.5 mmol) in 8 M-methanolic ammonia (150 ml) to stand at room temperature overnight. The products were then concentrated under reduced pressure, re-dissolved in chloroform (250 ml) and saturated aqueous ammonium chloride (250 ml) was added. The precipitated solid at the interface between the two layers was collected by filtration and suspended in distilled water (700 ml) at room temperature for 1 hour. The solid was filtered, washed with water (2 x 100 ml) and dried in vacuo over $P_2O_5$ to give the title compound (16.9 g, ca. 83%).

**6-(4-Chlorophenylthio)-9-(2,3-O-cyclopentylidene-β-D-ribofuranosyl)purine (*7*).**

1,1-Dimethoxycyclopentane (16.6 g, 0.128 mol) and toluene-4-sulphonic acid, monohydrate (0.81 g, 4.26 mmol) were added to a stirred solution of 6-(4-chlorophenylthio)-9-β-D-ribofuranosyl)purine (16.8 g, ca. 42.5 mmol) in dry tetrahydrofuran (250 ml) at room temperature. The reactants were heated at 50°C in an atmosphere of nitrogen for 1 hour, cooled to room temperature and then treated with triethylamine (0.7 ml, 5.0 mmol). The products were evaporated under reduced pressure and the residue was partitioned between chloroform (400 ml) and saturated aqueous sodium hydrogen carbonate (400 ml). The aqueous layer was back extracted with chloroform (2 x 100 ml), and the combined organic layers were dried ($MgSO_4$) and concentrated under reduced pressure. The residue obtained was fractionated by short column chromatography on silica gel. The appropriate fractions, eluted with $CHCl_3$-EtOH (100:0 - 99:1 v/v) were combined and evaporated under reduced pressure to give the title compound as a colourless foam (16.5 g, ca. 84%); $R_F$ 0.37 (system A), 0.30 (system B); $\delta_H$ [$(CD_3)_2SO$] 1.55 - 1.80 (6H, m), 1.98 (2H, m), 3.54 (2H, m), 4.29 (1H, m), 4.93 (1H, dd, J 2.3, 6.2 Hz), 5.11 (1H, t, J 5.2 Hz), 5.37 (1H, dd, J 2.5, 6.2 Hz), 6.25 (1H, d, J 2.4 Hz), 7.57 (2H, d, J 8.6 Hz), 7.66 (2H, d J 8.6 Hz), 8.62 (1H, s), 8.73 (1H, s); $\delta_C$ [$(CD_3)_2$ SO] 23.09, 23.65, 36.57, 36.71, 63.25, 81.85, 82.71, 85.93, 93.65, 123.73, 125.13, 129.64, 132.18, 136.21, 136.87, 143.01, 147.51, 151.67, 161.70.

**6-(4-Chlorophenylsulphonyl)-9-(2,3-O-cyclopentylidene-β-D-ribofuranosyl)purine (*8*).**

3-Chloroperbenzoic acid (ca. 55%, 32.0 g, ca. 0.10 mol) was added to a stirred solution of 6-(4-chlorophenylthio)-9-(2,3-O-cyclopentylidene-β-D-ribofuranosyl)purine (15.7 g, ca. 34 mmol) in dichloromethane (600 ml) at room temperature. After 3 hours, the products were diluted with dichloromethane (300 ml) and were then extracted with 5% aqueous sodium hydrogen sulphite (2 x 300 ml) and saturated sodium hydrogen carbonate (4 x 300 ml). The dried ($MgSO_4$) organic layer was concentrated under reduced pressure to give a colourless glass (15.1 g). Crystallisation of this material from absolute ethanol gave the title compound (10.8 g, ca. 65%) (Found: C, 51.4; H, 4.2; N, 11.1. $C_{21}H_{21}$ $ClN_4O_6S$ requires: C, 51.2; H, 4.3; N, 11.4%), m.p. 168°C; $R_F$ 0.28 (system B); $\delta_H$[$(CD_3)_2SO$] 1.55 - 1.80 (6H, m), 1.97 (2H, m), 3.55 (2H, m), 4.34 (2H, m), 4.91 (1H, dd, J 2.2, 6.2 Hz), 5.12 (1H, m), 5.37 (1H, dd, J 2.1, 6.2 Hz), 6.31 (1H, d, J 2.0 Hz), 7.77 (2H, d, J 8.5 Hz), 8.12 (2H, d, J 8.5 Hz), 9.03 (1H, s), 9.11 (1H, s); $\delta_C$ [$(CD_3)_2SO$] 23.10, 23.65, 36.49, 36.59, 63.11, 81.74, 83.44, 86.39, 93.16, 123.95, 129.66, 130.97, 131.10, 136.51, 141.58, 147.64, 151.57, 153.70, 154.91.

**6-(4-Chlorophenylsulphinyl)-9-(2,3-O-cyclopentylidene-β-D-ribofuranosyl)purine (*9*).**

A solution of 3-chloroperbenzoic acid (ca. 55%, 1.38 g, ca. 4.4 mmol) in dichloromethane (40 ml) was added dropwise over a period of 1 hour to a cooled (ice-salt bath, ca. -5°C), stirred solution of 6-(4-chlorophenylthio)-9-(2,3-O-cyclopentylidene-β-D-ribofuranosyl)purine (1.94 g, ca. 4.2 mmol) in dichloromethane (60 ml). The reactants were maintained at ca. -5°C for a further period of 2.5 hours and were then extracted with 5% aqueous sodium hydrogen sulphite (2 x 50 ml) and saturated aqueous sodium hydrogen carbonate (2 x 100 ml). The dried ($MgSO_4$) organic layer was concentrated under reduced pressure and the residue was fractionated by short column chromatography on silica gel. The appropriate fractions, eluted with $CHCl_3$-EtOH (99:1 v/v), were combined and evaporated under reduced pressure to give the title compound as a colourless foam (1.77 g, ca. 88%); $R_F$ 0.23 (system B); $\delta_H$ [$(CD_3)_2SO$] 1.55 - 1.80 (6H, m), 1.97 (2H, m), 3.55 (2H, m), 4.31 (1H, m), 4.91 (1H, m), 5.11 (1H, m), 5.36 (1H, m), 6.29 (1H, d, J 2.3 Hz), 7.62 (2H, m), 7.86 (2H, m), 8.95 (1H, s), 9.07 (1H, s); $\delta_C$ [$(CD_3)_2SO$] 23.07, 23.63, 36.51, 36.60, 63.08, 81.70, 81.73, 83.06, 83.15, 86.17, 86.21, 93.05, 93.17, 123.89, 123.92, 126.51, 129.74, 131.43, 131.48, 138.03, 140.66, 146.15, 146.20, 151.38, 151.44, 152.22, 152.24, 162.82, 162.84.

**Dibenzyl N-[9-(2,3-O-cyclopentylidene-β-D-ribofuranosyl)purin-6-yl]-L-aspartate (*11*).**

(a) N,N-Di-isopropylethylamine (3.40 ml, 19.5 mmol) was added to a stirred solution of 6-(4-chlorophenylsulphonyl)-9-(2,3-O-cyclopentylidene-β-D-ribofuranosyl) purine (2.40 g, ca. 4.9 mmol) and dibenzyl L-aspartate toluene-4-sulphonate (5.90 g, 12.15 mmol) in dry N,N-dimethylacetamide (50 ml) and the resulting mixture was heated at 100°C in an atmosphere of nitrogen. After 24 hours, the products were concentrated under reduced pressure and the residue was partitioned between chloroform (200 ml) and saturated aqueous sodium hydrogen carbonate (200 ml). The organic layer was dried ($MgSO_4$) and evaporated under reduced pressure. The residual yellow oil obtained was re-dissolved in ethyl acetate (150 ml) and the solution was extracted with cold 3 M-phosphoric acid (4 x 50 ml). The aqueous layer was back extracted with ethyl acetate (50 ml) and the combined organic layers were extracted with saturated aqueous sodium

hydrogen carbonate (100 ml), dried ($MgSO_4$) and concentrated under reduced pressure. The residual glass obtained was fractionated by short column chromatography on silica gel. The appropriate fractions, eluted with $CHCl_3$-EtOH (99:1 v/v), were combined and concentrated under reduced pressure to give the title compound as a pale yellow coloured glass (1.84 g, ca. 60%); $R_F$ 0.30 (system B); $\delta_H$ [$(CD_3)_2$ SO] 1.65 (4H, m), 1.74 (2H, m), 2.01 (2H, m), 3.10 (2H, m), 3.10 (2H, m), 3.56 (2H, m), 4.25 (1H, m), 4.93 (1H, dd, J 2.3, 6.2 Hz), 5.08 (2H, s), 5.11 (2H, s), 5.20 (1H, m), 5.31 (1H, m), 6.17 (1H, d, J 2.7 Hz), 7.29 (10H, m), 8.25 (1H, s), 8.35 (1H, m), 8.42 (1H, s); $\delta_C$ [$CDCl_3$] 23.08, 23.66, 36.60, 36.75, 49.79, 63.36, 66.84, 67.48, 81.98, 82.54, 85.75, 93.76, 121.52, 123.43, 128.20, 128.36, 128.52, 135.20, 135.32, 140.16, 147.92, 152.28, 154.16, 170.39, 170.62.

(b) N,N-Di-isopropylethylamine (1.40 ml, 8.0 mmol) was added to a stirred solution of 6-(4-chlorophenylsulphinyl)-9-(2,3-O-cyclopentylidene-β-D-ribofuranosyl)purine (0.954 g, ca. 2.0 mmol) and dibenzyl L-aspartate toluene-4-sulphonate (2.40 g, 4.9 mmol) in dry dimethylacetamide (20 ml), and the resulting mixture was heated at 70 to 75°C in an atmosphere of nitrogen. After 28 hours, the products were worked up and purified as in section (a) above to give the title compound (0.88 g, ca. 70%), identical (t.l.c., $^1H$ and $^{13}C$ n.m.r.) to the material obtained in section (a) above.

**N-[9-(β-D-ribofuranosyl)purin-6-yl]-L-aspartic acid (3).**

(a) Dibenzyl N-[9-(2,3-O-cyclopentylidene-β-D-ribofuranosyl)purin-6-yl]-L-aspartate [0.88 g, ca.1.4 mmol; prepared from 6-(4-chlorophenylsulphinyl)-9-(2,3-O-cyclopentylidene-β-D-ribofuranosyl)purine] was dissolved in formic acid - water (3:2 v/v; 30 ml) and the solution was stirred at room temperature. After 3 hours, water (30 ml) was added and the products were extracted with chloroform (4 x 30 ml). The combined organic layers were extracted aqueous sodium hydrogen carbonate (50 ml), and were then dried ($MgSO_4$) and concentrated under reduced pressure. The residue obtained was fractionated by short column chromatography on silica gel. The appropriate fractions, eluted with $CHCl_3$-EtOH (96:4 v/v), were combined and evaporated under reduced pressure to give a colourless glass (0.65 g); $R_F$ 0.22 (system A).

A solution of the latter material (0.65 g) in ethanol - acetic acid - water (8:1:1 v/v; 30 ml) and 10% palladium on activated carbon (0.13 g) were stirred together in an atmosphere of hydrogen at room temperature and atmosphereic pressure for 2 hours. The products were then filtered and the residual catalyst was washed with ethanol - water (1:1 v/v; 20 ml). The combined filtrate and washings were evaporated under reduced pressure, and the residue was crystallised from aqueous ethanol to give the title compound (0.38 g, ca. 68% overall yield for the two steps) (Found: C, 42.1; H, 4.7; N, 17.2. $C_{14}H_{17}N_5O_8.H_2O$ requires: C, 41.9; H, 4.8; N, 17.45%); m.p. 160°C (dec.); $[\alpha]_D^{20}$ - 48°C(c 1.6, $MeCONMe_2$); $R_T$ 5.3 min [0.1 M - aqueous triethylammonium acetate - MeCN (97:3 v/v)], 12.0 min [0.1 M - aqueous triethylammonium acetate - MeCN (98:2 v/v)]; $\lambda_{max}$ (95% EtOH) 267 (ε 18 200), $\lambda_{min}$ 232 (ε 2 000) nm; $\lambda_{max}$ (0.097 M - aqueous NaOH) 268 (ε 19 400), $\lambda_{min}$ 233 (ε 2 550) nm; $\delta_H$ [$(CD_3)_2SO$] 2.90 (2H, m), 3.57 (1H, m), 3.69 (1H, m), 3.97 (1H, m), 4.16 (1H, m), 4.61 (1H, m), 5.09 (1H, m), 5.19 (1H, d, J 4.2 Hz), 5.38 (1H, m), 5.48 (1H, d, J 5.8 Hz), 5.91 (1H, d, J 6.1 Hz), 7.96 (1H, d, J 7.9 Hz), 8.25 (1H, s), 8.41 (1H, s); $\delta_C$ [$(CD_3)_2SO$] 35.88, 49.52, 62.65, 70.65, 73.60, 85.92, 88.07, 119.90, 140.29, 148.66, 152.06, 154.05, 172.13, 172.89.

(b) Dibenzyl N-[9-(2,3-O-cyclopentylidene-β-D-ribofuranosyl)purin-6-yl]-L-aspartate [0.94 g, ca.1.5 mmol; prepared from 6-(4-chlorophenylsulphonyl)-9-(2,3-O-cyclopentylidene-β-D-ribofuranosyl)purine was treated with formic acid - water (3:2 v/v; 30 ml) as above, and the products were worked up and chromatographed to give a colourless glass (0.67 g). A solution of a portion of the latter material (0.28 g) in ethanol - acetic acid - water (8:1:1 v/v; 15 ml) and 10% palladium on activated carbon (0.06 g) were stirred together in an atmosphere of hydrogen at room temperature and atmospheric pressure for 2 hours. The products were then worked up as above to give the title compound (0.152 g, ca. 61% overall yield for the two steps), identical to material described in section (a) above.

**N-[9-(β-D-ribofuranosyl)purin-6-yl]-D,L-aspartic acid.**

Dibenzyl N-[9-(2,3-O-cyclopentylidene-β-D-ribofuranosyl)purin-6-yl]-D,L-aspartate was prepared as above by allowing 6-(4-chlorophenylsulphonyl)-9-(2,3-O-cyclopentylidene-β-D-ribofuranosyl)purine (0.52 g, 1.05 mmol), dibenzyl D,L-aspartate (0.83 g, 2.65 mmol), and N,N-di-isopropylethylamine (0.28 ml, 1.6 mmol) to react together in dry N,N-dimethylacetamide (10 ml) solution at 100°C for 24 hours. Following the removal of the cyclopentylidene and benzyl protecting groups from a large portion (0.32 g) of this material by the procedures described above in the prepartion of the L-aspartic acid enantiomer, the title substance [0.13g, ca. 36% overall yield based on (8)] was obtained as a colourless solid; $[\alpha]_D^{20}$ -31° (c 1.6, $MeCONMe_2$); $R_T$ 6.07 (ca. 50%), 8.15 (ca. 50%) min [0.1 M - aqueous triethylammonium acetate - acetonitrile (97:3 v/v)]. Under the same elution

conditions, pure crystalline N-[9-(β-D-ribofuranosyl)purin-6-yl]-L-aspartic acid has $R_T$ 6.07 min, and the mother liquors remaining after the crystallisation of the latter compound [prepared from 6-(4-chlorophenylsulphonyl)-9-(2,3-O-cyclopentylidene-β-D-ribofuranosyl)purine (8)] have $R_T$ 6.07 (ca. 66%) and 8.15 (ca. 33%) min.

**Ammonium salt of {N-[9-(β-D-ribofuranosyl)purin-6-yl]-L-aspartic acid} 5'-phosphate [Adenylosuccinic acid] (*1*).**

(a) N-Chlorosuccinimide (0.50 g, 3.74 mmol) was added to a stirred solution of dibenzyl phosphonate (1.00 g, 3.8 mmol) in dry benzene (15 ml) in an atmosphere of nitrogen at room temperature. After 90 minutes, the products were filtered and the filtrate was concentrated under reduced pressure at 10 to 15°C to give dibenzyl phosphorochloridate as a colourless oil. The latter material was added by syringe to a stirred solution of dry [following evaporation from pyridine (2 x 8 ml) solution] dibenzyl N-[9-(2,3-O-cyclopentylidene-β-D-ribofuranosyl)purin-6-yl]-L-aspartate [0.80 g, ca.1.27 mmol, prepared from 6-(4-chlorophenylsulphinyl)-9-(2,3-O-cyclopentylidene-β-D-ribofuranosyl)-purine] in anhydrous pyridine (8 ml) at ca. -40°C [acetone - dry ice bath] in an atmosphere of nitrogen. After two hours, the reaction mixture was removed from the cooling bath and saturated aqueous sodium hydrogen carbonate (20 ml) was added. The products were then concentrated under reduced pressure and the residual syrup was partitioned between chloroform (100 ml) and saturated aqueous sodium hydrogen carbonate (100 ml). The aqueous layer was back extracted with chloroform (30 ml), and the combined, dried ($MgSO_4$) organic layers were evaporated under reduced pressure. The residue obtained was fractionated by short column chromatography on silica gel. The appropriate fractions, eluted with $CHCl_3$-EtOH (99:1 v/v) were combined and evaporated under reduced pressure to give a pale yellow glass (1.03 g); $R_F$ 0.24 (system B); $\delta_P$ ($CDCl_3$) -0.2.
The latter material (1.03 g) was dissolved in formic acid - water (4:1 v/v; 25 ml) and the solution was allowed to stand at room temperature. After 3 hours, water (25 ml) was added and the products were extracted with chloroform (4 x 30 ml). The combined organic layers were washed with saturated aqueous sodium hydrogen carbonate (50 ml), dried ($MgSO_4$) and concentrated under reduced pressure. The residue obtained was fractionated by short column chromatography on silica gel. The appropriate fractions, eluted with $CHCl_3$-EtOH (97:3 v/v), were combined and evaporated under reduced pressure to give a colourless glass (0.734 g); $R_F$ 0.36 (system A), 0.13 (system B); $\delta_P$ ($CDCl_3$) -0.08.
A solution of this material (0.734 g) in ethanol - water (4:1 v/v; 50 ml) and 10% palladium on activated carbon (0.15 g) were stirred together in an atmosphere of hydrogen at room temperature and atmospheric pressure for 2 hours. The products were then filtered and the residual catalyst was washed with ethanol - water (1:1 v/v; 25 ml). Concentrated aqueous ammonia (d 0.88) was added to the combined filtrate and washings until the pH rose to ca. 9.0. The resulting solution was then evaporated under reduced pressure. The residual yellow coloured glass obtained was re-dissolved in water (10 ml) and the solution was heated at 60°C with activated charcoal for 30 minutes. The charcoal was removed by filtration and the filtrate was concentrated under reduced pressure to ca. 3 ml. Addition of the latter solution dropwise with stirring to absolute ethanol (ca. 300 ml) gave the title compound as a colourless precipitate (0.37 g, ca. 66% overall yield for the three steps); $R_T$ 7.4 (ca. 99.5%), 5.9 (ca. 0.5%) min [0.1 M- triethylammonium acetate - acetonitrile (98:2 v/v)]; $\delta_H$ ($D_2O$) 2.88 (1H, dd, J 7.7, 16.1 Hz), 2.97 (1H, dd, J 4.2, 16.0 Hz), 4.05 (2H, m), 4.30 (1H, m), 4.42 (1H, t, J 4.4 Hz), 4.66 (1H, t, J 5.4 Hz), 6.07 (1H, d, J 5.6 Hz), 8.21 (1H, s), 8.38 (1H, s); $\delta_C$ ($D_2O$) 39.65, 54.01, 65.17, 71.12, 75.17, 84.67 (d, J 8.3 Hz), 87.92; 119.74; 140.30, 148.94, 153.11, 154.41, 178.51, 178.84; $\delta_P$ ($D_2O$) 1.13.
(b) Dibenzyl N-[9-(2,3-O-cyclopentylidene-ß-D-ribofuranosyl)purin-6-yl]-L-aspartate [2.417 g, ca. 3.84 mmol, prepared from 6-(4-chlorophenylsulphonyl)-9-(2,3-O-cyclopentylidene-β-D-ribofuranosyl)purine was phosphorylated with dibenzyl phosphorochloridate [prepared from dibenzyl phosphonate (3.54 g, 13.5 mmol)] in pyridine (30 ml) solution under the conditions described above in section (a). The reaction mixture was worked up and fractionated in the same way and the product obtained was treated as above with formic acidwater (4:1 v/v) to give, after work up and chromatography, a colourless glass (1.98 g). A large portion (1.68 g) of the latter material was hydrogenated in the presence of palladized charcoal. Work up and neutralisation of the products with concentrated aqueous ammonia gave the title compound which was isolated as above as a colourless precipitate (1.024 g, ca. 59% overall yield for the three steps; $R_T$ 7.5 (ca. 91%), 5.9 (ca. 9%) min [0.1 M-triethylammonium acetate - acetonitrile (98:2 v/v)].

**Enzyme-promoted dephosphorylation of impurity in adenylosuccinic acid (1), prepared from 6-(4-chlorophenylsulphonyl)-9-(2,3-O-cyclopentylidene-β-D-ribofuranosyl)purine (*8*).**

The low retention time ($R_T$ 5.9 min) impurity in the ammonium salt of adenylosuccinic acid obtained in section (b) above was isolated by preparative l.c. A solution of the latter material (1.0 $A_{260}$ unit) and bacterial alkaline phosphatase (2.0 units) in 0.05 M-tris hydrochloride buffer (pH 7.5, 0.2 ml) was maintained at 25°C. After 15 minutes, complete digestion of the substrate to a product with $R_T$ 8.38 min [0.1 M-aqueous triethylammonium acetate - acetonitrile (97:3 v/v)] had occurred. The product had the same $R_T$ as the high $R_T$ component of N-[9-(β-D-ribofuranosyl)purin-6-yl]-D,L-aspartic acid (see above).

**Digestion of adenylosuccinic acid (*1*) with adenylosuccinate lyase.**

A solution of adenylosuccinate lyase (0.1 units) in 0.05 M-potassium phosphate buffer (pH 7.0, 0.1 ml) was added to a solution of ammonium adenlyosuccinate (0.0002 g) in the same buffer (0.2 ml) at 25°C. After 17 hours, the substrate ($R_T$ 10.15 min [0.1 M-aqueous triethylammonium acetate - acetonitrile (98.6 : 1.4 v/v)]) was completely converted into adenosine 5'-phosphate ($R_T$ 8.65 min).

## Claims

1. A method of synthesis of a substituted-amine derivative of a nucleoside or nucleotide wherein the substituent amino group is introduced by a nucleophilic displacement reaction between a sulphone or sulphoxide substituted heterocyclic compound forming or to form the heterocyclic moiety of said derivative and an alpha-amino acid or other reactive amino compound.

2. Preparation of adenosylosuccinic acid or adenylosuccinic acid according to claim 1, wherein the succinate group is attached to the purine ring by displacement reaction between L-aspartic acid and a purine carrying a sulphoxide or sulphone group at the 6-position.

3. Preparation according to claim 2, wherein during the displacement reaction the purine carries a 9-(β-D-furanosyl) group.

4. Preparation of adenylosuccinic acid according to claim 3, wherein subsequently to attachment of the succinate group the furanosyl group is phosphorylated at the 5-position.

5. Preparation according to any preceding claim, wherein the sulphoxide- or sulphone-substituted heterocyclic compound has itself been prepared by oxidation of the corresponding thio-derivative.

6. Preparation according to claim 5, wherein the thio-derivative has been prepared by reaction of a thiol with said heterocyclic compound as a 6-chloro or other reactive derivative.

7. Use of adenylosuccinic acid and/or adenosylosuccinic acid prepared as above in treatment or in the preparation of medicaments therefor, the indicated conditions being weakness of skeletal, cardiac or smooth muscles, particularly in the muscular dystrophies, in cardiac disorders associated with myocardial weakness or failure, and in other disorders associated with muscle weakness such as diabetes and chronic fatigue syndrome or post-viral fatigue syndrome and in disorders of the urinary and gastrointestinal tract and other smooth muscle systems; also degenerative and other diseases of the central and peripheral nervous system such as multiple sclerosis, Alzheimer's disease, other dementias, and impaired intellectual development or intellectual deterioration from any cause, and peripheral neuropathies of any cause.